# EUROPEAN PATENT SPECIFICATION

(11) **EP 1 178 736 B1**
(45) Date of publication and mention of the grant of the patent: **18.02.2004**
(21) Application number: 00926985.3
(22) Date of filing: 17.04.2000
(51) Int. Cl.: A23K 1/16, A61K 31/05, A61P 1/12

(54) **A COMPOSITION CONTAINING CARVACROL AND THYMOL FOR USE AS A BACTERICIDE**
CARVACROL UND THYMOL ENTHALTENDE ZUSAMMENSETZUNG ZUR VERWENDUNG ALS BAKTERIZIDE
COMPOSITION CONTENANT DU CARVACROL ET DU THYMOL UTILISEE COMME BACTERICIDE

(30) Priority: 12.05.1999 SE 9901733
(43) Date of publication of application: 13.02.2002
(73) Proprietor: AKZO NOBEL N.V., 6800 SB Arnhem (NL)
(72) Inventor: LOSA, Riccardo, CH-1145 Bière (CH)
(74) Representative: Andersson, Rolf (SE)
(86) International application number: PCT/EP2000/003513
(87) International publication number: WO 2000/069277

(56) References cited:
- EP-A- 0 630 577
- EP-A- 0 904 780
- WO-A-96/37210
- WO-A-97/01348
- WO-A-99/59430
- M. TIZIANA BARATTA ET AL.: "Chemical composition, antimicrobial and antioxidative activity of laurel, sage, rosemary, oregano and coriander essential oils" JOURNAL OF ESSENTIAL OIL RESEARCH., vol. 10, no. 6, 1998, pages 618-627, XP000929938 XX, XX ISSN: 1041-2905
- DATABASE CAB [Online] CAB INTERNATIONAL, WALLINGFORD, OXON, GB; Accession Nr. 83:13485, TYUTYUNNIK, V.I. ET AL.: "The composition and bactericidal properties of Monarda fistulosa essential oil" XP002144692 & TRUDY VNII EFIROMASLICHNYCKH KULT'UR, vol. 14, 1982, pages 15-21,
- CHEMICAL ABSTRACTS, vol. 131, no. 23, 6 December 1999 (1999-12-06) Columbus, Ohio, US; abstract no. 308815u, LIN, WENQUIN ET AL.: "Studies on the chemical constituents of essential oil and its antibacterial effect on Mosla chinensis Maxim growing in Fujian Province" page 284; column 2; XP002144691 & FUJIAN SHIFAN DAXUE XUEBAO, ZIRAN KEXUEBAN, vol. 15, no. 2, 1999, pages 88-91,
- N. DIDRY ET AL.: "Activité antibactérienne du thymol, du carvacrol et de l'aldéhyde cinnamique seuls ou associés" PHARMAZIE., vol. 48, no. 4, 1993, pages 301-304, XP002144690 VEB VERLAG VOLK UND GESUNDHEIT. BERLIN., DD ISSN: 0031-7144

## Description

The present invention relates to a composition containing the natural substances carvacrol and thymol. The composition exhibits a synergistic bactericidal effect against Treponema, which causes severe muco-hemorrhagic diseases, such as swine dysentery and severe diseases affecting the hoofs of hoofed animals, particularly cloven-hoofed animals, such as ruminants. The composition is suitably administered to an animal via a diet composition, drinking water or a drench bath.

Diseases caused by Treponema are common in animal stocks. For example, an anaerobic spirochete, Treponema hyodysenteriae, is considered to be the primary etiologic agent of swine dysentery (SD). See for instance D.L. Harris and R.J. Lysons (1992), Swine dysentery, in: Diseases of swine, 7^{th} edition, Iowa State University Press, Ames, Iowa, USA. SD is a severe muco-hemorrhagic diarrhoea. Pigs actually affected with SD usually consume very low amounts of feed causing an essential reduction in growth and a considerable economical loss. In Top Agrar (1998), Vol. 4, page 52 it is reported that in Germany one third of the piglets herds and as much as one half of the growing pig herds probably are Treponema-positive. Large efforts are therefore justified to prevent the diseases to be spread. It is also well known that Treponema causes severe diseases affecting the hoofs of hoofed animals, particularly cloven-hoofed animals, such as ruminants.

M. Tiziana Baratta et al.: "Chemical composition, antimicrobial and antioxidative activity of laurel, sage, rosemary, oregano and coriander essential oils" Journal of Essential Oil Research., vol. 10, no 6, 1998, pages 618-627, XP000929938 XX, XX ISSN: 1041-2905, disclose the chemical composition of five essential oils, derived from sage, rosemary, oregano, laurel and coriander, and also the antibacterial activity of these essential oils against a number of microorganisms.

WO 96/37210 describes a pharmaceutical composition wherein the active agent is an extract from certain plants, especially origanum vulgaris, Thymus vulgaris and Menta piprita. In general, the extracted oil contains 3% thymol and 60-70% carvacrol

Furthermore, in WO 97/01348 it is disclosed a pharmaceutical composition comprising a herbal essential oil containing thymol and carvacrol as its main ingredients and pharmaceutically acceptable carrier. The total amount of thymol and carvacrol in said essential oil is at least 55%, preferably 70% by weight of said essential oil, and the ratio of carvacrol to thymol is at least 10. This pharmaceutical composition can be used in the prevention and treatment of coccidiosis in poultry.

A wide spectrum of antibiotics, such as streptomycin, bacitracin, neomycin, tylosin, gentamycin, chlostetracycline, virginamycin and lincomycin, have been reported to be effective in the treatment of SD. Where the disease is endemic, preventive medication is often added to the animal feed. However, it. seems that the antibiotics become less and less effective and Top Agrar (1998), Vol. 4, page 52, reports a resistance ratio of Treponema of 99% to tylosin, 92% to lyncomycin and 48% to tiamulin in 1997.

However, in recent years there has been an intense debate about the use of chemical and antibiotic growth promoters and in many countries a ban on this type of feed additives is being considered. Thus, there is an urgent need for agriculture to develop substances which are in line with reliable and generally accepted practice and not of a medicinal nature.

One objective of the present invention is to provide natural substances as active agents, which are suitable for the administration to the animal via a diet, drinking water or a drench bath for the cure, prevention or alleviation of the diseases caused by Treponema. Another objective is to reduce the negative effects on the growth.

According to the invention it has been found that a composition, containing the natural substances carvacrol in an amount of 5 ppm to 90% by dry weight and thymol in an amount of 5 ppm to 80% by dry weight, in a weight ratio between 1:5 and 10:1, preferably between 2:3 and 4:1, exhibits a synergistic effect against Treponema, and thereby diminishing the negative effect these bacteriae have on the health and growth of animals. According to the invention said composition can be used as an active agent for the manufacture of a medicament for treatment of diseases caused by Treponema. By the expression "a natural substance" is in this context understood a substance which consists of compounds occurring in nature and that is obtained from natural products or through synthesis. The composition is usually administered as a diet composition or as a drinking water containing carvacrol and thymol in amounts of 5-2000 ppm, preferably 20-600 ppm, calculated on the dry weight of the diet composition including nutritive substances or on the weight of the drinking water. The composition may also be present in an amount of 0.2-30% by weight in a drench bath for the treatment of the hoofs of hoofed animals, particularly cloven-hoofed animals, such as ruminants. The invention also includes a premix and a diet additive that may be used in the preparation of the diet composition as well as a drinking water supplement and a drench bath supplement.

The composition and the diet composition may also contain other natural substances which enhance the health and improve the growth. In the diet composition these substances are normally present in an amount of 0.1 to 30 ppm, calculated on the dry weight. Examples of such substances and their amounts are 1-5 ppm guaiacol, 1-5 ppm eugenol, 0.1-2 ppm capsascin and 1-20 mg tannin. Other suitable ingredients in the diet composition are flavourings of natural substances. They are usually present in an amount of 0.2-50 ppm, calculated on the dry weight of the diet composition. Examples of suitable flavourings and their amounts are 0.05-0.5 ppm creosol, 0.1-5 mg anethole, 0.1-2 ppm of deca-, undeca- and/or dodecalactones, 0.1-2 quinoleine, 0.1-2 ppm ionones and/or irone, 0.05-1 ppm gingerol, 0.05-2 ppm piperine, C.05-1 ppm propylidene and/or butylidene phtalides and 0.1-5 ppm amyl and/or benzyl salicylate.

The incorporation of active ingredients into the diet composition is usually carried out by preparing a premix of the active compounds carvacrol and thymol and other suitable additives. Such a premix may contain 1-10% by dry weight of carvacrol and thymol, 0-40% by dry weight of growth improving additives, flavourings and health enhancing additives, and 50-99% by weight of an absorbing support. The support may contain, for example, 40-50% by weight of wood fibres, 8-10% by weight of stearin, 4-5% by weight of curcuma powder, 4-5% by weight of rosemary powder, 22-28% by weight of limestone, 1-3% by weight of a gum, such as gum arabic, 5-50% by weight of sugar and/or starch and 5-15% by weight of water.

This premix can then be mixed with common feed components, such as vitamins, enzymes, mineral salts, ground cereals, protein-containing components, carbohydrate-containing components, wheat middlings and/or brans in the preparation of a diet composition additive which contains 0.2-5% by weight of the premix. The diet composition additive is then finally added to the diet composition in such quantities that the feed will contain 5-2000 ppm, preferably 20-600 ppm, of the active mixture. The diet composition additive normally constitutes 0.3-3.5% by weight of diet composition.

The diet composition according to the invention usually contains, calculated on the dry weight of the feed, the following ingredients:
a) 0-80%, preferably 10-70%, by weight of cereals,
b) 0-30%, preferably 1-12%, by weight of fat,
c) 0-85%, preferably 10-50%, by weight of protein containing nutritious substances of a type other than cereals, and
d) 1-500 ppm, preferably 10-100 ppm, of the mixture. The total amounts of a)-d) are preferably at least 80% by weight.

When preparing the diet composition, the diet composition additive can be mixed with the dry ingredients consisting of cereals, such as ground or crushed wheat, oats, barley, maize and rice; vegetable protein feed based on e.g. rapeseed, soya bean and sunflower; animal protein feed, such as blood meal, meat and bone meal and fish meal; molasses; and milk products, such as various milk powders and whey powders. After mixing all the dry additives, the liquid ingredients and ingredients, which after heating become liquid, can be added. The liquid ingredients may consist of lipids, such as fat, for example slaughter fat and vegetable fat, optionally liquefied by heating, and/or of carboxylic acids, such as a fatty acid. After thorough mixing, a mealy or particulate consistency is obtained, depending on the degree of grinding of the ingredients. To prevent separation during storage, water should preferably be added to the animal feed, which then is subjected to a conventional pelletising, expanding or extruding process. Any excess water can be removed by drying. If desired, the resulting granular animal feed can also be crushed to a smaller particle size.

The drinking water supplement may contain 2-90% by dry weight, preferably 10-50% by dry weight, of carvacrol and thymol. Beside carvacrol and thymol the supplement also contains 10-98% by dry weight of a large number of other ingredients. Common ingredients are mineral salts, vitamines, natural substances enhancing the health and growth, flavourings, water-soluble or water-dispersable carriers, such as sugars, powdered milk, milk-by-products and cellulose derivatives, dispersing agents and stabilisers, such as water-soluble or water-dispersable polymers. Suitable examples of natural substances enhancing the health and growth have earlier been described. When preparing the drinking water, the supplement is normally added to the water in such an amount that the concentration of the natural substance becomes 5-2000 ppm, preferably 20-600 ppm.

The drench bath supplement may contain 30-98% by dry weight of carvacrol and thymol and 2-70% by weight of mineral salts, water-soluble or water-dispersable carriers, dispersing agents and/or stabilisers, such as water-soluble or water-dispersable polymers.

Within the scope of the invention, it is also possible to produce a suspension of the diet composition. This is especially convenient if the feed is prepared for immediate consumption.

The present invention will now be further illustrated by the following Examples.

### Example 1

The antimicrobial activity of the composition of the invention towards Treponema innocens and Triponema hyodysenteriae was determined in vitro. In the tests the following organisms, growth media, culture conditions and evaluation method were used.

| | |
|---|---|
| Organisms | Treponema innocens, ATTC 29796 |
| | Treponema hyodysenteriae, ATCC 31212 |
| | |
| Growth media | Caseine-peptone soymeal-peptone agar USP (Caso-Agar, Merch No. 5458) + 5% Sheep blood |
| | |
| Culture conditions | Anaerobic incubation at 37°C for 4-6 days |
| | |
| Evaluation method | Agar dilution test (according to DIN 58940, teil 6) |

Agar plates were prepared by using the growth media, to which 10% by weight of a solution of carvacrol and/or thymol in polypropylene glycol had been added.

Cell suspensions with a concentration of 10.9 cfu/ml were prepared of each of the organisms. The single suspensions were then distributed on the agar surface using a Multipoint inoculator applying 1µl to a final surface of about 0.5 cm². For every concentration of carvacrol and/or thymol two parallel plates were inoculated and on each plate three inoculation points of each of the two organisms are applied. After the inoculation period the growth of the organisms were observed. If no growth was observed, the concentration of carvacrol and/or thymol was in the next test reduced to half. The minimum concentration of carvacrol and/or thymol leading to a total suppression of bacterial growth is noted as the MIC value (minimal inhibitory concentration) of the active components or compounds. The following results were obtained.

**Table 1.**

| **Minimal inhibitory concentration** | | | |
|---|---|---|---|
| **Test No**. | **Active compound** | **MIC value, ppm** | |
| | | **Treponema innocens** | **Treponema hyodysenteriae** |
| 1 | Thymol | 625 | 625 |
| 2 | Carvacrol | 313 | 313 |
| 3 | 2/3 Thymol | 156 | 156 |
| | 1/3 Carvacrol | | |
| 4 | 1/2 Thymol | 156 | 156 |
| | 1/2 Carvacrol | | |
| 5 | 1/3 Thymol | < 78 | < 78 |
| | 2/3 Carvacrol | | |

From the results it is evident that the composition of the invention exhibits a synergistic antimicrobial effect on the tested organisms.

### Example 2

In order to determine the efficacy of the mixture of the invention to reduce the occurrence of Treponema some tests were performed with grower pigs (25 kg to 100 kg), which were put on commercial diets with the following composition.

| | |
|---|---|
| Crude protein, % | 24.0 |
| Crude fat, % | 6.0 |
| Crude fiber, % | 3.5 |
| Crude ash, % | 5.0 |
| Lysine, % | 1.35 |
| MJDE, kg | 14.3 |

This diet was formulated by mixing suitable amounts of wheat, lupin kernel, canola meal, rice pollard, meat meal, blood meal and tallow. To the diet administrated to the experimental group 100 ppm of a mixture containing 67 ppm carvacrol and 33 ppm thymol were added.

During the growing phase animal faeces were controlled for presence of Treponema and development of dysentery was recorded. The following results were obtained.

**Table 2.**

| **Occurence of swine dysentery and Treponema in faeces** | | | |
|---|---|---|---|
| | **No. of animals controlled** | **Presence of Treponema in faeces** | **Development of swine dysentery** |
| Control | 23 | 8 | 1 |
| Experimental | 10 | 0 | 0 |

These results clearly indicate that the occurrence of Treponema is much lower when the mixture of the invention is added to the feed in comparison to a negative control.

### Example 3

The purpose of the following experiments were to investigate the effect of the mixture according to the present invention to increase animal growth and inhibition of the growth of Treponema. Forty male pigs in two pens were allocated to each treatment. The pigs were weighed individually at an age of 46 days, when the experimental diets were introduced. The experiment continued for 42 days. All the diets in the tests contained the following basic composition.

| | |
|---|---|
| Crude protein, % | 24.4 |
| Crude fat, % | 6.3 |
| Crude fiber, % | 3.5 |
| Crude ash, % | 5.3 |
| Lysine, % | 1.35 |
| MJDE, kg | 14.5 |

This composition was formulated by mixing suitable amounts of wheat, lupin kernel, canola meal, rice pollard, meat meal, blood meal and tallow.

In a control test A 100 ppm Olaquindox™ and 25 ppm Tiamulin™ had been added to the basic composition while in tests I and II according to the invention the diets contained 100 ppm and 200 ppm of the mixture of carvacrol and thymol disclosed in Example 2. In a control test B the diet consisted of the basic composition. The following results were obtained.

**Table 3.**

| **Effect on the performance of pigs between 46 and 67 days of age.** | | | | |
|---|---|---|---|---|
| **Tests** | **A** | **B** | **I** | **II** |
| Start weight (kg) | 15.3 | 15.2 | 15.3 | 15.2 |
| Final weight (kg) | 28.6 | 27.0 | 26.9 | 27.2 |
| Daily gain (g) | 666 | 588 | 578 | 595 |
| Feed intake (g/d) | 1100 | 1000 | 933 | 971 |
| Feed gain | 1.65 | 1.70 | 1.61 | 1.63 |

**Table 4.**

| **Effects on the performance of pigs during 67 to 88 days of age**. | | | | |
|---|---|---|---|---|
| **Tests** | **A** | **B** | **I** | **II** |
| Final weight (kg) | 42.8 | 41.9 | 40.8 | 41.2 |
| Daily gain (g) | 665 | 695 | 655 | 671 |
| Feed intake (kg/d) | 1.38 | 1.42 | 1.32 | 1.37 |
| Feed gain | 2.04 | 2.05 | 2.02 | 2.05 |

From these results one can clearly see that the addition of the mixture of the invention increases the growth especially in the period between 46 and 67 days of age.

The effect of diets containing the active mixture of the invention and the presence of Treponema in faeces was investigated by feeding four groups with five pigs in each group on the diets used in control test A and B and in tests I and II. The faeces of each pig was examined in regard to the presence of Treponema after the pigs have been fed on the diets 4, 8 and 16 days. The following results were obtained.

**Table 3.**

| **Occurence of Treponema in faeces** | | | | |
|---|---|---|---|---|
| **Time, days** | **Samples with Treponema** | | | |
| | **Control A** | **Control B** | **Test I** | **Test II** |
| 4 | 1 | 3 | 4 | 4 |
| 8 | 3 | 3 | 0 | 0 |
| 16 | 0 | 0 | 0 | 0 |

From the results it is evident that the pigs fed on the diet according to the invention did not have Treponema in their faeces after 8 days while the pigs fed on the diets in Controls A and B needed treatment for 16 days.

## Claims

1. Use of a composition containing carvacrol in an amount of 5 ppm to 90% by dry weight, and thymol in an amount of 5 ppm to 80% by dry weight, the weight ratio between carvacrol and thymol being from 1:5 to 10:1, as an active agent for the manufacture of a medicament for treatment of diseases caused by Treponema.

2. Use according to claim 1, where the weight ratio is from 2:3 to 4:1.

3. Use according to claim 1 or 2, where the medicament is a diet composition containing carvacrol and thymol in amounts from 5 to 2000 ppm.

4. Use according to any one of claims 1-3 for the treatment of swine dysentery or for the treatment of the hoofs of hoofed animals.

5. A diet composition, **characterized in that** it contains carvacrol and thymol in amounts of 5-2000 ppm calculated on dry weight of the diet composition, the weight ratio between carvacrol and thymol being 2:3 to 4:1.

6. A diet composition according to claim 5, **characterized in that** the diet composition contains 0.1-30 ppm calculated on the dry weight of natural substances, selected from the group consisting of guaiacol, eugenol, capsaicin and tannin, which enhance the health and increase the growth, and/or 0.2-50 ppm calculated on the dry weight of natural substances, selected from the group consisting of creosol, anethole, deca-, undeca- and/or dodecalactones, quinoleine, ionones and/or irone, gingerol, piperine, propylidene and/or butylidene phtalides, amyl and/or benzyl salicylate.

7. A drinking water supplement, **characterized in that** it contains 2-90% by dry weight of carvacrol and thymol, the weight ratio between carvacrol and thymol being 2:3 to 4:1.

8. A drench bath supplement, **characterized in that** it contains 30-98% by dry weight of carvacrol and thymol, the weight ratio between carvacrol and thymol being 2:3 to 4:1.

## Patentansprüche

1. Verwendung einer Zusammensetzung enthaltend Carvacrol in einer Menge von 5 ppm bis 90%, bezogen auf das Trockengewicht, und Thymol in einer Menge von 5 ppm bis 80%, bezogen auf das Trockengewicht, wobei das Gewichtsverhältnis zwischen Carvacrol und Thymol 1:5 bis 10:1 beträgt, als Wirkstoff zur Herstellung eines Arzneimittels zur Behandlung von Krankheiten, die durch Treponema verursacht werden.

2. Verwendung nach Anspruch 1, worin das Gewichtsverhältnis 2:3 bis 4:1 beträgt.

3. Verwendung nach Anspruch 1 oder 2, worin das Arzneimittel eine diätetische Zusammensetzung ist, die Carvacrol und Thymol in Mengen von 5 bis 2.000 ppm enthält.

4. Verwendung nach irgendeinem der Ansprüche 1 bis 3 zur Behandlung von Schweinedysenterie oder zur Behandlung der Hufe von Huftieren.

5. Diätetische Zusammensetzung, **dadurch gekennzeichnet, dass** sie Carvacrol und Thymol in Mengen von 5 bis 2.000 ppm, berechnet auf das Trockengewicht der diätetischen Zusammensetzung, enthält, wobei das Gewichtsverhältnis zwischen Carvacrol und Thymol 2:3 bis 4:1 beträgt.

6. Diätetische Zusammensetzung nach Anspruch 5, **dadurch gekennzeichnet, dass** die diätetische Zusammensetzung 0,1 bis 30 ppm, berechnet auf das Trockengewicht, von Naturstoffen ausgewählt aus der Gruppe bestehend aus Guajacol, Eugenol, Capsaicin und Tannin, welche die Gesundheit fördern und das Wachstum steigern, und/oder 0,2 - 50 ppm, berechnet auf das Trockengewicht, von Naturstoffen ausgewählt aus der Gruppe bestehend aus Kreosol, Anethol, Deca-, Undeca- und/oder Dodecalactonen, Chinolin, lononen und/ oder Iron, Gingerol, Piperin, Propyliden- und/oder Butylidenphthaliden, Amyl- und/ oder Benzylsalicylat enthält.

7. Trinkwasserzusatz, **dadurch gekennzeichnet, dass** er 2 bis 90%, bezogen auf das Trockengewicht, Carvacrol und Thymol enthält, wobei das Gewichtsverhältnis zwischen Carvacrol und Thymol 2:3 bis 4:1 beträgt.

8. Tränkbadzusatz, **dadurch gekennzeichnet, dass** er 30 bis 98%, bezogen auf das Trockengewicht, Carvacrol und Thymol enthält, wobei das Gewichtsverhältnis zwischen Carvacrol und Thymol 2:3 bis 4:1 beträgt.

## Revendications

1. Utilisation d'une composition contenant une quantité de carvacrol de 5 parties par million (ppm) jusqu'à 90% du poids sec, et une quantité de thymol de 5 ppm jusqu'à 80% du poids sec, le rapport en poids du carvacrol au thymol étant de 1 : 5 à 10 : 1, comme agent actif pour la fabrication d'un médicament pour le traitement des maladies provoquées par le tréponème.

2. Utilisation selon la revendication 1, dans laquelle le rapport en poids est de 2 : 3 à 4 : 1.

3. Utilisation selon la revendication 1 ou 2, dans laquelle le médicament est une composition alimentaire contenant du carvacrol et du thymol dans des quantités allant de 5 à 2000 ppm.

4. Utilisation selon l'une quelconque des revendications 1-3 pour le traitement de la dysenterie porcine ou pour le traitement des sabots d'animaux ongulés.

5. Composition alimentaire, **caractérisée en ce qu'**elle contient du carvacrol et du thymol dans des quantités de 5-2000 ppm calculées par rapport au poids sec de la composition alimentaire, le rapport en poids du carvacrol au thymol étant de 2 :3 à 4 : 1.

6. Composition alimentaire selon la revendication 5, **caractérisée en ce que** la composition alimentaire contient de 0,1-30 ppm calculées par rapport au poids sec de substances naturelles, sélectionnées dans le groupe constitué par le guaïacol, l'eugénol, la capsaïcine et le tannin, qui améliorent la santé et augmentent la croissance, et/ou 0,2 - 50 ppm calculées par rapport au poids sec de substances naturelles, sélectionnées dans le groupe constitué par le créosol, l'anéthole, les déca-, les undéca- et/ou les dodécalactones, la quinoléine, les ionones et/ou l'irone, le gingérol, la pipérine, le propylidène et/ou les phtalides de butylidène, le salicylate d'amyle et/ou de benzyle.

7. Supplément pour eau de boisson, **caractérisé en ce qu'**il contient de 2 - 90% du poids sec est constitué de carvacrol et de thymol, le rapport en poids du carvacrol par rapport au thymol étant de 2 : 3 à 4 : 1.

8. Supplément pour le bain de trempage, **caractérisé en ce qu'**il contient 30 - 98% en poids sec de carvacrol et de thymol, le rapport en poids du carvacrol au thymol étant de 2 : 3 à 4 : 1.
